# EUROPEAN PATENT SPECIFICATION

(11) **EP 3 500 531 B1**
(45) Date of publication and mention of the grant of the patent: **17.03.2021**
(21) Application number: 17751372.8
(22) Date of filing: 07.08.2017
(51) Int. Cl.: C02F 1/52, G01N 15/04, B01D 21/32, G01N 1/20

(54) **TEST APPARATUS FOR A WASTE WATER TREATMENT SYSTEM**
PRÜFVORRICHTUNG FÜR EINE ABWASSERAUFBEREITUNGSANLAGE
APPAREIL DE TEST POUR SYSTÈME DE TRAITEMENT DES EAUX USÉES

(30) Priority: 18.08.2016 GB 201614133
(43) Date of publication of application: 26.06.2019
(73) Proprietor: Cdenviro Limited, Cookstown, County Tyrone BT80 8DG (GB)
(72) Inventor: DOBBS, Sean, Cookstown County Tyrone BT80 9DG (GB)
(74) Representative: FRKelly
(86) International application number: PCT/EP2017/069975
(87) International publication number: WO 2018/033423

(56) References cited:
- EP-A1- 3 045 891
- CH-A- 516 329
- KR-B1- 101 131 500
- US-A- 4 279 759
- US-A1- 2013 335 731

## Description

### FIELD OF THE INVENTION

This invention relates to a waste water treatment system having a test apparatus in particular for testing the correct dosage of a flocculating agent added to waste water to be passed into a settling tank.

### BACKGROUND OF THE INVENTION

Waste water streams containing entrained solid impurities and contaminants in suspension are produced from numerous quarrying, mining, chemical or industrial processes. It is often desirable to reuse such water, particularly in regions prone to water shortages.

Before the waste water can be re-used, the solid impurities and contaminants (referred to as "fines") must be removed from the water. This is typically done by passing the water into a settling tank wherein the fines are able to settle out under the action of gravity. A flocculating agent is typically added to the waste water to which the fines bind to bring them out of suspension.

The amount of flocculating agent added to the waste water needs to be accurately metered to ensure efficient separation of the fines from the waste water without wastage of the relatively expensive flocculating agent. It is therefore desirable to periodically test the settlement rate of the fines in the waste water to determine that the correct amount of flocculating agent is present.

This testing is typically achieved by withdrawing waste water from the settlement tank into a vertically arranged transparent section of pipework defining a test chamber, trapping a sample of the waste water in the test chamber and timing how long it takes for the solid material within the sample in the test chamber to fall out of suspension, typically by using a photo-detector mounted at a predetermined height on the test chamber.

In a known arrangement waste water is withdrawn under gravity from the settlement tank to flow through the test chamber before passing into a collection sump. When it is desired to carry out a test, valves upstream and downstream of the test chamber are closed and a timer is started to determine the time interval before the photo-detector detects clear water at said predetermined height in the test chamber. Typically once a test has been completed, the downstream valve is opened (keeping the upstream valve closed) and a further valve is opened to pass clean water through the test chamber to flush the test chamber. The valves are then shut to close off the test chamber and waste water collected in the collection sump is pumped back up into the settlement tank.

A problem with such known systems is that the height difference between the test chamber and the settlement tank can be significant, resulting in a high flow rate through the test chamber when both the upstream and downstream valves are open. When the upstream and downstream valve are then suddenly closed the sudden change in flow velocity within the test chamber can create turbulent conditions, breaking up flocculated fines in the test sample and resulting in a slower than representative settlement rate. The additional pipework, pumps and sump also takes up space and adds to the cost of the system. US 2013/336731, EP 3045891, US 4279759, KR101131500 and CH516329 disclose known test devices suitable for waste water testing.

### SUMMARY OF THE INVENTION

According to the present invention there is provided a waste water treatment system as claimed in claim 1.

In a preferred embodiment a first sensor may be provided at a first height, preferably adjacent the top of the test chamber, and a second sensor is provided at a second height, below said first height and preferably adjacent a lower end of the test chamber, the timer being started when the first sensor detects clear water and stopped when the second sensor detects clear water. The or each sensor may comprise a photo-detector detecting the transmission of light through the waste water within the test chamber. The inlet of the fluid passageway may be located below the outlet such that waste water flows upwardly through the test chamber from the inlet to the outlet. The flow path may comprise an upwardly extending pipe.

Preferably said inlet extends tangentially from said bend in the flow path such that waste water preferentially enters the inlet due to the inertia of the waste water.

The wall adjacent the outlet opening of the fluid passageway may comprise a semi-circular wall extending around an upstream side of the outlet opening around which wall the waste water in the flow path is constrained to flow.

At least a portion of said test chamber may be formed from a transparent material.

A clean water inlet may be provided for supplying clean water to an upstream end of the test chamber under the control of a further valve for flushing the test chamber following said test..

In one embodiment the system may comprise a mixing chamber within which a flocculating agent is mixed with a waste water stream and a settlement tank downstream of said mixing chamber wherein fines settle out from said waste water, said flow path comprises a pipeline extending from the mixing chamber to the settlement tank.

The flow path preferably extends in an upwardly direction from said mixing chamber to said settlement tank. The test chamber may extend substantially parallel to said flow path between said inlet and said outlet of the fluid passageway.

### BRIEF DESCRIPTION OF THE DRAWINGS

A waste water treatment system in accordance with an embodiment of the present invention will now be described, by way of example only, with reference to the accompanying drawings, in which :-
Figure 1 is a perspective view of a waste water treatment system in accordance with an embodiment of the present invention; and

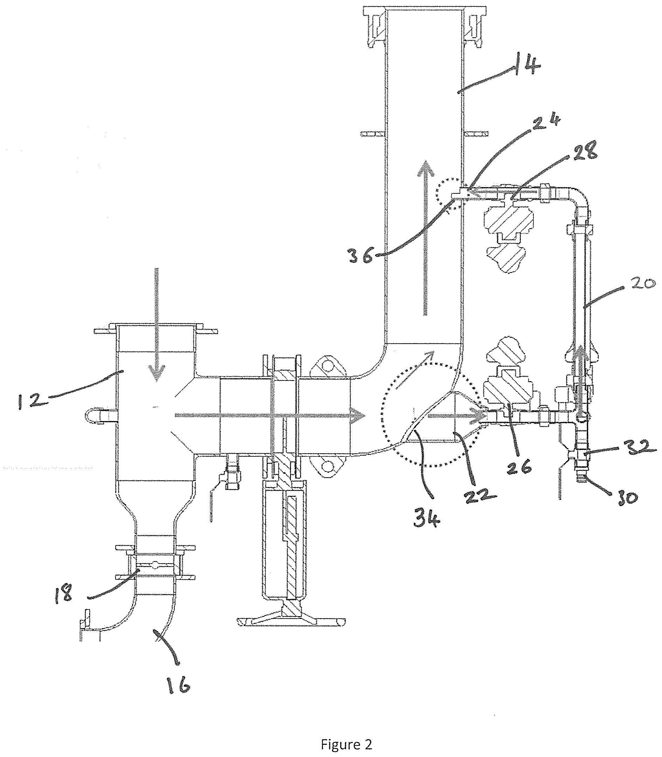

Figure 2 is a sectional view through a part of the system of Figure 1 showing the test apparatus in more detail.

### DETAILED DESCRIPTION OF THE DRAWINGS

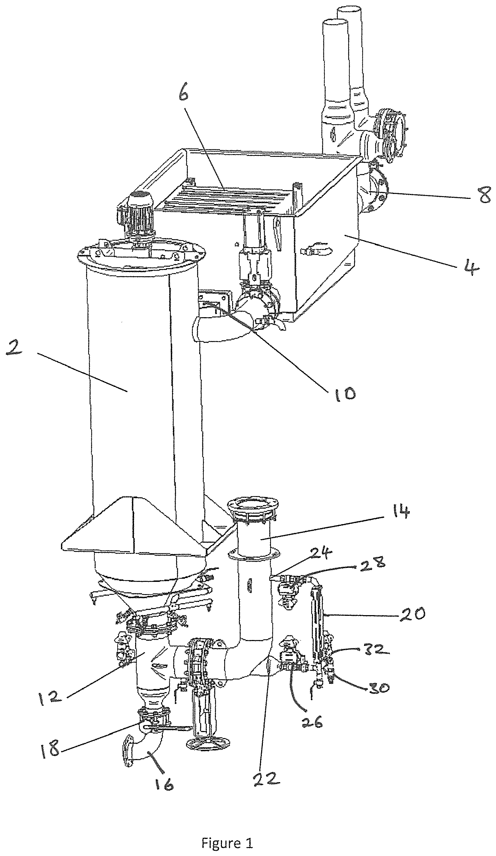

Figure 1 illustrates a water treatment system in accordance with an embodiment of the present invention. The waste water treatment system shown in Figure 1 comprises a dosing and mixing apparatus 2 within which a flocculating agent and/or other chemical is added to and mixed with a waste water stream upstream of a settling tank.

A de-aerating apparatus 4 is provided upstream of the mixing apparatus 2 to remove entrained air from the waste water. There is typically a lot of air entrained in waste water discharged from sand and aggregate washing and grading processes and other washing processes and this entrained air inhibits the fast and effective settlement of flocculated solids. By removing air from the waste water prior to addition of the flocculating agent, the effectiveness of the flocculating agent is enhanced, potentially allowing the use of a smaller settlement tank.

The de-aerating chamber 4 incorporates a dividing wall 6 dividing the chamber 4 into first and second regions, an the inlet 8 communicating with the first region of the chamber 4 and an outlet 10 communicating with the second region of the chamber 4, the dividing wall 6 defining a weir over which waste water has to pass to travel from the inlet 8 to the outlet 10 of the chamber 4. This has the effect of reducing turbulence in the waste water.

The waste water, to which a predetermined dose of a flocculating agent has been added, passes out of a lower end 12 of the mixing apparatus 2 and passes up a vertically arranged delivery pipe 14 to be delivered into a settlement tank (not shown). A drain 16 is provided below the lower end 12 of the mixing apparatus 2, said drain 16 being normally closed by a shut off valve 18.

A test chamber 20, defined by a vertically arranged transparent pipe, is arranged in parallel to the delivery pipe 14, having an inlet 22 at a lower end of the delivery pipe 14 and an outlet 24 adjacent an upper end of the delivery pipe 14, said outlet 24 being arranged perpendicular to the delivery pipe 14. The test chamber 20 may be use to test the rate of settlement of the fines out of suspension as a test of the dosage of flocculating agent within a sample. At least one light source may be provided for directing light through the test chamber and a respective photo-detector may be provided for determining the turbidity of the sample contained therein by receiving light from the respective light source once it has passed through the waste water in the test chamber 20. The light source and photo-detector may be provided at a predetermined height on the test chamber 20, whereby the water at said height becomes clear when settlement of the fines within the sample is complete. The light source and detector may operate in the infra-red range. In a preferred embodiment a first detector is provided at a first height, preferably adjacent the top of the test chamber 20, and a second detector is provided at a second height, below said first height and preferably adjacent a lower end of the test chamber 20.

A first valve 26 is provided for controlling communication between the inlet 22 and the test chamber 20 and a second valve 28 is provided for controlling communication between the test chamber 20 and the outlet 24. A further valve 32 is provided for controlling communication between a fresh water supply 30 and the lower end of the test chamber 20 for flushing the test chamber 20, as will be described in more detail below.

The inlet 22 leading from the delivery pipe 14 into the test chamber 20 is arranged in a high pressure region of the delivery pipe 14, more specifically on the outside of a right angle bend 34 at the lower end of the delivery pipe, the inlet 22 extending tangentially from an outer side of the bend 34 where the velocity and pressure of the waste water is at a maximum.

The shape of the outlet 24 is such that a low pressure region is generated within the delivery pipe 14 in the vicinity of the outlet 24. This is achieved by providing a curved wall 36 extending into the delivery pipe 14 on an upstream side of the outlet 24 relative to the normal direction of flow of waste water within the delivery pipe 14, said wall 36 shielding the outlet 24 from the flow within the delivery pipe 14 and creating a low pressure region at the outlet 24 as the water flows around the wall 36.

The pressure differential between the inlet 22 and outlet 24 causes water to preferentially flow upwardly through the test chamber 20 when the first and second valves 26,28 are both open as waste water flows upwardly through the delivery pipe 14.

During normal operation of the waste water treatment system the first and second valves 26,28 and the further valve 32 remain closed and waste water passes from the mixing apparatus 2, through the outlet 12 and up the delivery pipe 14 to be delivered into the settlement tank.

Periodically, under the control of a PLC, the valve 26,28 are opened to initiate a test cycle. Once the first and second valves 26 and 28 are opened, a portion of the waste water flows through into the inlet 22, through the test chamber 20 and returns into the delivery pipe 14 due to the pressure differential between the inlet 22 and outlet 24, as discussed above. After a predetermined period of time the first and second valves 26,28 are then closed to trap a sample of waste water and entrained fines within the test chamber 20.

The fines begin to settle out of the waste water within the test chamber 20 and collect in the bottom of the test chamber 20. A timer may be started when the first detector detects clear water and stopped when the second detector detects clear water. The period of time before the second photo-detector detects clear water is indicative of the correct dosage of flocculating agent.

Once the test cycle has completed the first valve 26 remains closed while the second valve 28 is opened and the further valve 32 is opened to supply fresh water into the lower end of the test chamber 20 to flush the test chamber 20, the settled fines and the flushing water passing into the delivery pipe 14 via the outlet 24. Once the flushing process has completed after a predetermined period of time the further and second valves 28,32 are closed, once again isolating the test chamber 20 from the delivery pipe 14 until the next test cycle is initiated.

The test apparatus of the water treatment system according to the present invention requires no sump or pump or associated level sensors or pipework and is considerably more compact than prior art systems. The test chamber 20 is never empty (either containing waste water from the delivery pipe 14 or clear water from the fresh water supply 30. Therefore the liquid within the test chamber acts as a damper when the first and second valves 26,28, are first opened, avoiding turbulence and resulting in minimal to no flocculated solids break up. The fact that the flow of waste water through the test chamber 20 occurs in parallel to the delivery pipe 14, waste water being sampled from and returned to the deliver pipe 14, provides more gentle and slower flow rates through the test apparatus, maintaining floc structure and avoiding sudden pressure changes and associated turbulence, allowing faster test cycle times compared to prior art systems, where delays have to be built in to allow turbulence to settle.

The invention is not limited to the embodiment described herein but can be amended or modified without departing from the scope of the present invention as defined by the appended claims.

## Claims

1. A waste water treatment system incorporating a flow path (14) having a bend (34) and a test apparatus (2), said test apparatus (2) comprising a fluid passageway having an inlet (22) communicating with a first region of said flow path (14) of the waste water treatment system and an outlet (24) communicating with a second region of said flow path (14), downstream of said first region, at least a portion of said fluid passageway defining a test chamber (20) within which a test may be carried out, wherein first and second valves (26,28) are provided in the fluid passageway upstream and downstream of the test chamber (20), said first and second valves (26,28) being operable to trap a test sample of waste water within the test chamber (20) during a test process, said test chamber (20) being arranged substantially vertically, at least one sensor being associated with the test chamber (20) for sensing the turbidity of the waste water within the test chamber (20) at a predetermined level therein, a controller incorporating a timer being provided for determining the time taken for settlement of fines out of suspension in the waste water trapped within the test chamber (20) when the first and second valves (26,28) are closed as determined by the detection of an absence of or reduced level of turbidity at said predetermined level by said at least one sensor as a measure of the amount of a flocculating agent in the waste water sample, **characterised in that** the inlet (22) of the fluid passageway communicates with the flow path (14) at an outer side of said bend (34) in the flow path (14) and wherein the outlet (24) of the fluid passageway in the flow path (14) includes an outlet opening in the flow path and a wall (36) extending into the flow path (14) on an upstream side of the outlet opening, said wall (26) generating a low pressure region adjacent the outlet opening such that a pressure differential exists between said inlet (22) and outlet (24) whereby waste water is caused to be diverted from the flow path (14) through the test chamber from the inlet to the outlet of the fluid passageway.

2. A system as claimed in claim 1, wherein a first sensor is provided at a first height, adjacent the top of the test chamber (20), and a second sensor is provided at a second height, below said first height and adjacent a lower end of the test chamber (20), the timer being started when the first sensor detects clear water and stopped when the second sensor detects clear water.

3. A system as claimed in claim 1 or claim 2, wherein said at least one sensor comprises a photo-detector detecting the transmission of light through the waste water within the test chamber (20).

4. A system as claimed any preceding claim, wherein the inlet (22) of the fluid passageway is located below the outlet (24).

5. A system as claimed in claim 4, wherein said flow path (14) comprises an upwardly extending pipe.

6. A system as claimed in any preceding claim, wherein said inlet (22) extends tangentially from said bend (34) in the flow path (14).

7. A system as claimed in any preceding claim, wherein said wall (36) comprises a semi-circular wall extending around an upstream side of the outlet opening.

8. A system as claimed in any preceding claim, wherein at least a portion of said test chamber (20) is formed from a transparent material.

9. A system as claimed in any preceding claim further comprising a water inlet (30) adapted to supply clean water to an upstream end of the test chamber (20) under the control of a further valve (32) to flush the test chamber following said test.

10. A system as claimed in any preceding claim, comprising a mixing chamber (2) and a settlement tank downstream of said mixing chamber, said flow path comprising a pipeline (14) extending from the mixing chamber (2) to the settlement tank.

11. A system as claimed in claim 10, wherein said flow path extends in an upwardly direction from said mixing chamber (2) to said settlement tank.

12. A system as claimed in claim 11, wherein said test chamber (20) extends substantially parallel to said flow path (14) between said inlet (22) and said outlet (24) of the fluid passageway.

## Patentansprüche

1. Abwasserbehandlungssystem, das einen Strömungsweg (14) mit einer Biegung (34) und eine Prüfvorrichtung (2) einbezieht, wobei die Prüfvorrichtung (2) einen Fluiddurchgang umfasst, der einen mit einem ersten Bereich des Strömungswegs (14) des Abwasserbehandlungssystems in Verbindung stehenden Einlass (22) und einen mit einem zweiten Bereich des Strömungswegs (14) stromabwärts des ersten Bereichs in Verbindung stehenden Auslass (24) umfasst, wobei mindestens ein Abschnitt des Fluiddurchgangs eine Prüfkammer (20) definiert, in der eine Prüfung durchgeführt werden kann, wobei ein erstes und ein zweites Ventil (26, 28) in dem Fluiddurchgang stromaufwärts und stromabwärts der Prüfkammer (20) bereitgestellt sind, wobei das erste und das zweite Ventil (26, 28) betätigbar sind, um eine Prüfprobe von Abwasser während eines Prüfvorgangs in der Prüfkammer (20) einzuschließen, wobei die Prüfkammer (20) im Wesentlichen vertikal angeordnet ist, wobei mindestens ein Sensor mit der Prüfkammer (20) assoziiert ist, um die Trübheit des Abwassers in der Prüfkammer (20) auf einem vorgegebenen Niveau darin zu erfassen, wobei eine Steuereinheit, die einen Zeitmesser einbezieht, dazu bereitgestellt ist, die Zeit zu bestimmen, die das Absetzen von Feinstoffen aus der Suspension in dem in der Prüfkammer (20) eingeschlossenen Abwasser dauert, wenn das erste und das zweite Ventil (26, 28) geschlossen sind, was durch das Erkennen einer Abwesenheit von oder eines verringerten Niveaus an Trübheit auf dem vorgegebenen Niveau durch den mindestens einen Sensor als Maß der Menge eines Flockungsmittels in der Abwasserprobe bestimmt wird, **dadurch gekennzeichnet, dass** der Einlass (22) des Fluiddurchgangs mit dem Strömungsweg (14) an einer Außenseite der Biegung (34) in dem Strömungsweg (14) in Verbindung steht, und wobei der Auslass (24) des Fluiddurchgangs in dem Strömungsweg (14) eine Auslassöffnung in dem Strömungsweg und eine sich auf einer stromaufwärtigen Seite der Auslassöffnung in den Strömungsweg (14) erstreckende Wand (36) umfasst, wobei die Wand (26) einen der Auslassöffnung benachbarten Niederdruckbereich erzeugt, sodass eine Druckdifferenz zwischen dem Einlass (22) und dem Auslass (24) vorliegt, wodurch bewirkt wird, dass Abwasser von dem Strömungsweg (14) durch die Prüfkammer von dem Einlass zu dem Auslass des Fluiddurchgangs umgeleitet wird.

2. System nach Anspruch 1, wobei ein erster Sensor auf einer ersten Höhe, der Oberseite der Prüfkammer (20) benachbart bereitstellt ist und ein zweiter Sensor auf einer zweiten Höhe, unter der ersten Höhe und einem unteren Ende der Prüfkammer (20) benachbart bereitgestellt ist, wobei der Zeitmesser gestartet wird, wenn der erste Sensor klares Wasser erkennt, und gestoppt wird, wenn der zweite Sensor klares Wasser erkennt.

3. System nach Anspruch 1 oder Anspruch 2, wobei der mindestens eine Sensor einen Fotodetektor umfasst, der die Übertragung von Licht durch das Abwasser in der Prüfkammer (20) erkennt.

4. System nach einem der vorangehenden Ansprüche, wobei der Einlass (22) des Fluiddurchgangs unter dem Auslass (24) liegt.

5. System nach Anspruch 4, wobei der Strömungsweg (14) ein sich nach oben erstreckendes Rohr umfasst.

6. System nach einem der vorangehenden Ansprüche, wobei sich der Einlass (22) tangential von der Biegung (34) in dem Strömungsweg (14) erstreckt.

7. System nach einem der vorangehenden Ansprüche, wobei die Wand (36) eine halbkreisförmige Wand umfasst, die sich um eine stromaufwärtige Seite der Auslassöffnung erstreckt.

8. System nach einem der vorangehenden Ansprüche, wobei mindestens ein Abschnitt der Prüfkammer (20) aus einem durchsichtigen Material gebildet ist.

9. System nach einem der vorangehenden Ansprüche, ferner umfassend einen Wassereinlass (30), der dazu angepasst ist, einem stromaufwärtigen Ende der Prüfkammer (20) unter Kontrolle eines weiteren Ventils (32) sauberes Wasser zuzuführen, um die Prüfkammer nach der Prüfung zu spülen.

10. System nach einem der vorangehenden Ansprüche, umfassend eine Mischkammer (2) und einen Absetzbehälter stromabwärts der Mischkammer, wobei der Strömungsweg eine Rohrleitung (14) umfasst, die sich von der Mischkammer (2) zu dem Absetzbehälter erstreckt.

11. System nach Anspruch 10, wobei sich der Strömungsweg in einer Aufwärtsrichtung von der Mischkammer (2) zu dem Absetzbehälter erstreckt.

12. System nach Anspruch 11, wobei sich die Prüfkammer (20) im Wesentlichen parallel zu dem Strömungsweg (14) zwischen dem Einlass (22) und dem Auslass (24) des Fluiddurchgangs erstreckt.

## Revendications

1. Système de traitement des eaux usées incorporant un chemin d'écoulement (14) ayant un coude (34) et un appareil de test (2), ledit appareil de test (2) comportant un passage de fluide ayant une entrée (22) en communication avec une première région dudit chemin d'écoulement (14) du système de traitement des eaux usées et une sortie (24) en communication avec une deuxième région dudit chemin d'écoulement (14), en aval par rapport à ladite première région, au moins une partie dudit passage de fluide définissant une chambre de test (20) à l'intérieur de laquelle un test peut être effectué, dans lequel des première et deuxième vannes (26, 28) sont mises en œuvre dans le passage de fluide en amont et en aval par rapport à la chambre de test (20), lesdites première et deuxième vannes (26, 28) servant à piéger un échantillon de test d'eaux usées à l'intérieur de la chambre de test (20) au cours d'un processus de test, ladite chambre de test (20) étant agencée sensiblement à la verticale, au moins un capteur étant associé à la chambre de test (20) pour détecter la turbidité des eaux usées à l'intérieur de la chambre de test (20) à un niveau prédéterminé dans celle-ci, un dispositif de commande incorporant un temporisateur étant mis en œuvre pour déterminer le temps pris pour la décantation des matériaux fins en provenance de la suspension dans les eaux usées piégées à l'intérieur de la chambre de test (20) quand les première et deuxième vannes (26, 28) sont fermées tel qu'il est déterminé par la détection d'une absence ou d'un niveau réduit de turbidité audit niveau prédéterminé par ledit au moins un capteur comme mesure de la quantité d'un réactif de floculation dans l'échantillon d'eaux usées, **caractérisé en ce que** l'entrée (22) du passage de fluide communique avec le chemin d'écoulement (14) au niveau d'un côté extérieur dudit coude (34) dans le chemin d'écoulement (14) et dans lequel la sortie (24) du passage de fluide dans le chemin d'écoulement (14) comprend une ouverture de sortie dans le chemin d'écoulement et une paroi (36) s'étendant jusque dans le chemin d'écoulement (14) sur un côté en amont de l'ouverture de sortie, ladite paroi (26) générant une région basse pression adjacente par rapport à l'ouverture de sortie de telle sorte qu'une pression différentielle existe entre ladite entrée (22) et ladite sortie (24) ce par quoi les eaux usées sont amenées à être déviées du chemin d'écoulement (14) au travers de la chambre de test depuis l'entrée jusqu'à la sortie du passage de fluide.

2. Système selon la revendication 1, dans lequel un premier capteur est mis en œuvre à une première hauteur, de manière adjacente par rapport à la partie supérieure de la chambre de test (20), et un deuxième capteur est mis en œuvre à une deuxième hauteur, sous ladite première hauteur et de manière adjacente par rapport à une extrémité inférieure de la chambre de test (20), le temporisateur étant démarré quand le premier capteur détecte des eaux limpides et arrêté quand le deuxième capteur détecte des eaux limpides.

3. Système selon la revendication 1 ou la revendication 2, dans lequel ledit au moins un capteur comporte un photodétecteur qui détecte la transmission de lumière au travers des eaux usées à l'intérieur de la chambre de test (20).

4. Système selon l'une quelconque des revendications précédentes, dans lequel l'entrée (22) du passage de fluide est située sous la sortie (24).

5. Système selon la revendication 4, dans lequel ledit chemin d'écoulement (14) comporte un tuyau s'étendant vers le haut.

6. Système selon l'une quelconque des revendications précédentes, dans lequel ladite entrée (22) s'étend de manière tangentielle depuis ledit coude (34) dans le chemin d'écoulement (14).

7. Système selon l'une quelconque des revendications précédentes, dans lequel ladite paroi (36) comporte une paroi semi-circulaire s'étendant autour d'un côté en amont de l'ouverture de sortie.

8. Système selon l'une quelconque des revendications précédentes, dans lequel au moins une partie de ladite chambre de test (20) est formée à partir d'un matériau transparent.

9. Système selon l'une quelconque des revendications précédentes, comportant par ailleurs une entrée d'eau (30) adaptée pour fournir de l'eau propre à une extrémité en amont de la chambre de test (20) sous le contrôle d'une autre vanne (32) pour vidanger la chambre de test suite audit test.

10. Système selon l'une quelconque des revendications précédentes, comportant une chambre de mélange (2) et un réservoir de décantation en aval par rapport à ladite chambre de mélange, ledit chemin d'écoulement comportant une canalisation (14) s'étendant depuis la chambre de mélange (2) jusqu'au réservoir de décantation.

11. Système selon la revendication 10, dans lequel ledit chemin d'écoulement s'étend dans une direction allant vers le haut depuis ladite chambre de mélange (2) jusqu'audit réservoir de décantation.

12. Système selon la revendication 11, dans lequel ladite chambre de test (20) s'étend de manière sensiblement parallèle par rapport audit chemin d'écoulement (14) entre ladite entrée (22) et ladite sortie (24) dudit passage de fluide.
